(19) Europäisches Patentamt
European Patent Office
Office européen des brevets

(11) **EP 4 697 335 A1**

(12) **EUROPEAN PATENT APPLICATION**
published in accordance with Art. 153(4) EPC

(43) Date of publication:
**18.02.2026  Bulletin 2026/08**

(21) Application number: **23932598.8**

(22) Date of filing: **29.05.2023**

(51) International Patent Classification (IPC):
***G16B 20/10*** (2019.01)    ***G16B 30/10*** (2019.01)

(52) Cooperative Patent Classification (CPC):
Y02A 90/10

(86) International application number:
**PCT/CN2023/096747**

(87) International publication number:
**WO 2024/212318 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.04.2023  CN 202310439706**

(71) Applicant: **BGI Genomics Co., Limited
Guangdong 518083 (CN)**

(72) Inventors:
• **PENG, Jiguang
  Shenzhen, Guangdong 518083 (CN)**

• **XIANG, Jiale
  Shenzhen, Guangdong 518083 (CN)**
• **YE, Haodong
  Shenzhen, Guangdong 518083 (CN)**
• **SUN, Xiangzhong
  Shenzhen, Guangdong 518083 (CN)**
• **LI, Dongdong
  Shenzhen, Guangdong 518083 (CN)**
• **SUN, Juan
  Shenzhen, Guangdong 518083 (CN)**
• **PENG, Zhiyu
  Shenzhen, Guangdong 518083 (CN)**

(74) Representative: **Schwarz & Partner Patentanwälte GmbH
Patentanwälte
Wipplingerstraße 30
1010 Wien (AT)**

(54) **STRC GENE COPY NUMBER VARIATION DETECTION METHOD BASED ON WHOLE GENOME SEQUENCING**

(57)    An STRC gene copy number variation detection method based on whole genome sequencing, comprising: carrying out sequence comparison on an STRC gene and an STRCP1 gene to find out differential sites of the STRC gene and the STRCP1 gene; for each differential site, reading sequences of corresponding STRC positions and STRCP1 positions in a genome from a variation detection file; calculating the total copy number of true genes and pseudogenes by using a preset reference site in the genome as a benchmark; calculating an STRC gene copy ratio on each differential site; calculating the STRC gene copy number on each differential site according to the total copy number and the STRC gene copy ratio; and determining the STRC gene copy number on each exon according to the STRC gene copy number on each differential site. The method can implement measurement of the STRC copy number, simplifies a detection process, improves the detection throughput, and reduces costs.

performing a sequence alignment between a STRC gene and a STRCP1 gene to search out differentiated sites — S110

reading, from a variant calling file, for each of the differentiated sites, respective sequences at corresponding positions in the STRC gene and the STRCP1 gene in a genome — S120

calculating a total copy number of the true gene and the pseudo gene with a reference site preset in the genome as a benchmark — S130

calculating a STRC gene copy ratio for each of the differentiated sites — S140

calculating a first STRC gene copy number for each of the differentiated sites based on the total copy number and the STRC gene copy ratio — S150

determining a second STRC gene copy number for each exon based on the first STRC gene copy number for each of the differentiated sites — S160

Figure 2

**Description**

This application is based on and claims priority to Chinese Patent Application No. 202310439706.7, titled "STRC gene copy number variation detection method based on whole genome sequencing" and filed on April 12, 2023, the entire content of which is incorporated herein by reference.

## FIELD

The present disclosure relates to the technology field of data processing, and specifically relates to a method for detecting copy number variation of stereocilin (STRC) gene based on whole genome sequencing.

## BACKGROUND

Hereditary deafness is one of the major birth defects in China. The incidence of hearing loss in newborns of normal population is as high as 1~3‰. Among all causes of deafness, genetic factors account for 50 - 60%. It is known that more than 150 genes may cause deafness, posing a great challenge to clinical diagnosis for hereditary deafness due to the genetic heterogeneity. Hearing loss may be classified as mild, moderate, severe and extremely severe according to its degree, where mild to moderate deafness accounts for about 35% of sensorineural hearing loss cases, and is likely to be left out in routine neonatal screening. Several genes have been reported to be associated with mild to moderate deafness, including GJB2 gene and STRC gene. Deletions in the STRC gene present as the second most common genetic cause of mild to moderate hearing loss, only after the GJB2 gene, which causes most of genetic hearing loss. Such a hearing loss is of congenital phenotypes and moderate class, even after age 50. Therefore, the detection of a STRC gene copy number may be used to screen and diagnose congenital hearing loss in newborns.

However, STRC is highly homologous with a pseudogene, stereocilin pseudogene 1 (STRCP1), with 99.6% coding region and 98.9% intron region being highly similar, resulting in inaccurate detection of STRC through currently high-throughput sequencing. At present, quantitative PCR or multiplex ligation-dependent probe amplification (MLPA), or amplification on the complete true gene followed by Sanger sequencing, are the mainstream methods for STRC copy number detection, which however additionally increases the detection cost and experimental time.

## SUMMARY

In view of this, the present disclosure provides in embodiments a method for detecting copy number variation of stereocilin (STRC) gene based on whole genome sequencing (WGS), to solve the problem of the additionally increased detection cost and experimental time for current methods of STRC copy number detection.

In a first aspect, the present disclosure provides in embodiments a method for detecting copy number variation of a STRC gene based on whole genome sequencing, including: performing a sequence alignment between the STRC gene and a STRCP1 gene to search out differentiated sites; reading, from a variant calling file, for each of the differentiated sites, respective sequences at corresponding positions in the STRC gene and the STRCP1 gene in a genome; calculating a total copy number of the true gene and the pseudogene with a reference site preset in the genome as a benchmark; calculating a STRC gene copy ratio for each of the differentiated sites; calculating a first STRC gene copy number for each of the differentiated sites based on the total copy number and the STRC gene copy ratio; and determining a second STRC gene copy number for each exon based on the first STRC gene copy number for each of the differentiated sites.

In a second aspect, the present disclosure provides in embodiments a device for detecting copy number variation of a STRC gene based on whole genome sequencing, including: a differentiated-site searching module, configured to perform a sequence alignment between the STRC gene and a STRCP1 gene to search out differentiated sites; a sequence reading module, configured to read from a variant calling file, for each of the differentiated sites, respective sequences at corresponding positions in the STRC gene and the STRCP1 gene in a genome; a total copy number calculating module, configured to calculate a total copy number of the true gene and the pseudogene with a reference site preset in the genome as a benchmark; a first ratio calculating module, configured to calculate a STRC gene copy ratio for each of the differentiated sites; a first copy number calculating module, configured to calculate a first STRC gene copy number for each of the differentiated sites based on the total copy number and the STRC gene copy ratio; and a second copy number calculating module, configured to determine a second STRC gene copy number for each exon based on the first STRC gene copy number for each of the differentiated sites.

In a third aspect, the present disclosure provides in embodiments a terminal apparatus, including: one or more processors; and a memory, for storing one or more programs executable by said one or more processors; wherein said one or more processors are coupled with the memory, and said one or more programs is configured to execute the method for detecting copy number variation of a STRC gene based on whole genome sequencing according to embodiments of the first aspect.

In a fourth aspect, the present disclosure provides in embodiments a computer readable storage medium having stored therein a program code that, when executed by a processor, implements the method for detecting copy number variation of a STRC gene based on whole genome sequencing according to embodiments of the first aspect.

According to embodiments of the present disclosure, the method, device, terminal apparatus and computer readable storage medium as provided perform a sequence alignment between the STRC gene and the STRCP1 gene to search out differentiated sites; read from a variant calling file , for each of the differentiated sites, respective sequences at corresponding positions in the STRC gene and the STRCP1 gene in a genome; calculate a total copy number of the true gene and the pseudogene with a reference site preset in the genome as a benchmark; calculate a STRC gene copy ratio for each of the differentiated sites; calculate a first STRC gene copy number for each of the differentiated sites based on the total copy number and the STRC gene copy ratio; and determine a second STRC gene copy number for each exon based on the first STRC gene copy number for each of the differentiated sites.

According to embodiments of the present disclosure, the method for detecting copy number variation of STRC gene based on WGS could detect copy numbers of STRC for hereditary deafness, thereby expanding the range of genetic diseases detectable by WGS. Compared with traditional detection methods for highly homologous genes, the method is with simplified detection process, improved detection throughput, reduced detection cost and increased product competitiveness. Moreover, the method could automatically judge and output the copy number of each exon of STRC, which can be used for gene screening of healthy people or genetic etiology diagnosis of deaf patients.

## DESCRIPTION OF THE DRAWINGS

In order to illustrate technical solutions in embodiments of the present disclosure or in the prior art more clearly, drawings used in the description of the embodiments or the prior art are introduced briefly hereinafter. Apparently, the drawings described in the following text illustrate some embodiments of the present disclosure, other drawings may be obtained by those skilled in the art based on these drawings without any creative efforts.

Figure 1 shows application scenarios of a method for detecting copy number variation of STRC gene based on whole genome sequencing according to embodiments of the present disclosure;
Figure 2 is a block diagram illustrating a method for detecting copy number variation of STRC gene based on whole genome sequencing according to an embodiment of the present disclosure;
Figure 3 is a flow chart illustrating acquisition and screening of differentiated sites between STRC and STRCP1;
Figure 4 shows a correlation between copy numbers obtained by MLPA and by analyzing WGS data with a method for detecting copy number variation of STRC gene based on whole genome sequencing according to an embodiment of the present disclosure;
Figure 5 is a histogram illustrating a copy number distribution on STRC gene in 9,582 WGS samples according to an embodiment of the present disclosure;
Figure 6 is a block diagram illustrating a device for detecting copy number variation of STRC gene based on whole genome sequencing according to an embodiment of the present disclosure;
Figure 7 is a block diagram illustrating a terminal apparatus according to an embodiment of the present disclosure;
Figure 8 is a block diagram illustrating a computer readable storage medium according to an embodiment of the present disclosure.

## DETAILED DESCRIPTION

The technical solutions in the embodiments of the present disclosure will be described clearly and completely hereinafter in conjunction with the drawings of the embodiments of the present disclosure. Apparently, the described embodiments are only some of the embodiments of the present disclosure, rather than all embodiments. Any of other embodiments made by the person skilled in the art based on the embodiments in the present disclosure without any creative efforts, fall into the scope of the present disclosure.

A method for detecting copy number variation of STRC gene based on whole genome sequencing, and a device, a terminal apparatus, and a computer readable storage medium therefor provided by embodiments of the present disclosure will be described in detail with reference to drawings, to illustrate the present disclosure in more detail.

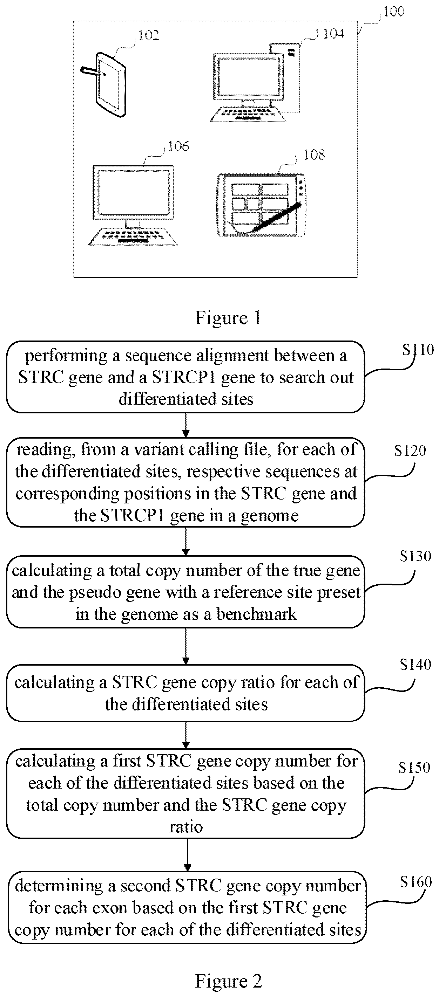

Referring to Figure 1, which shows application scenarios of the method for detecting copy number variation of STRC gene based on whole genome sequencing according to embodiments of the present disclosure, the application scenarios include a terminal apparatus 100 provided in the embodiment of the present disclosure. The terminal apparatus 100 may be various electronic devices (such as the diagrams for 102, 104, 106, and 108) having a display screen, including but not limited to smart phones and computer devices, in which the computer devices may be at least one of desktop computers, portable computers, laptop computers, tablet computers, and the like. The terminal apparatus 100 may generally refer to one of a plurality of terminal apparatuses, and the embodiment only takes the terminal apparatus 100 as an example. Those skilled in the art may know that the number of such terminal apparatuses may be greater or less. For example, there may be only a few terminal apparatuses, or dozens or hundreds of terminal apparatuses, or even more. The embodiment of the present disclosure is not limited by the number and type of the terminal apparatus. The terminal apparatus 100 may be used to execute the method for detecting copy number variation of STRC gene based on whole genome sequencing as provided in embodiments of the present disclosure.

In an optional embodiment, the application scenario may include a server in addition to the terminal apparatus 100 as provided in embodiments of the present disclosure, in which a network is provided between the server and the terminal apparatus. The network is used to provide a medium for providing communication links between the terminal apparatus and the server. The network may include various connection types, such as wired, wireless communication links, or fiber optic cables, among others.

It should be understood that the number of terminal apparatuses, networks, and servers is merely illustrative, and there may be any number of terminal apparatuses, networks, and servers, depending on implementation needs. For example, the server may be a server cluster composed of multiple servers. The terminal apparatus interacts with the server through the network to receive or send messages. The server may be a server that provides various services, and may be used to execute the steps of the method for detecting copy number variation of STRC gene based on WGS according to embodiments of the present disclosure. In addition, when the terminal apparatus executes the method for detecting copy number variation of STRC gene based on WGS according to embodiments of the present disclosure, a part of the steps may be executed by the terminal device, and another part of the steps may be executed by the server, which is not limited herein.

Based on this, provided in embodiments of the present disclosure is a method for detecting copy number variation of stereocilin (STRC) gene based on whole genome sequencing. Referring to Figure 2, which is a block diagram illustrating the method for detecting copy number variation of STRC gene based on whole genome sequencing according to an embodiment of the present disclosure, reference will be made to the method applied to the terminal apparatus as shown in Figure 1, where the method includes the following steps S110-S160.

At step S110, the STRC gene and STRCP1 gene are subject to a sequence alignment to search out differentiated sites.

Specifically, STRC gene, i.e. stereocilin gene, encodes a protein associated with the hair bundles of sensory hair cells in the inner ear. Hair bundles are composed of stiff microvilli called stereo cilium and are related to the mechanosensation of sound waves. This gene is part of a tandem copy of chromosome 15, a second copy being a pseudogene. Mutations in this gene cause autosomal recessive nonsyndromic deafness. STRCP1 gene is a homologous gene of STRC gene, having highly similar coding region and intron region therewith reaching 99.6% and 98.9% respectively. At present, the two genes cannot be distinguished by high-throughput sequencing as commonly used, and it is therefore difficult to calculate the copy number of STRC gene. Yet, the embodiments of the present disclosure provide the method, with WGS, to analyze STRC gene and STRCP1 gene in a sequenced sample (i.e., sequencing results of a sample to be sequenced), to determine the copy number of STRC gene.

Firstly, sequences of STRC gene and STRCP1 gene are obtained and then aligned, so as to determine differentiated sites between the two genes.

In an embodiments of the present disclosure, in the step S110, performing a sequence alignment between the STRC gene and STRCP1 gene to search out differentiated sites includes: extracting from a human reference genome STRC gene and STRCP1 gene; performing the sequence alignment between STRC gene and STRCP1 gene to obtain all of initially differentiated sites between the STRC gene and the STRCP1 gene; and selecting from all of the initially differentiated sites the differentiated sites with a population frequency less than a first preset threshold and a coefficient of variation less than a second preset threshold.

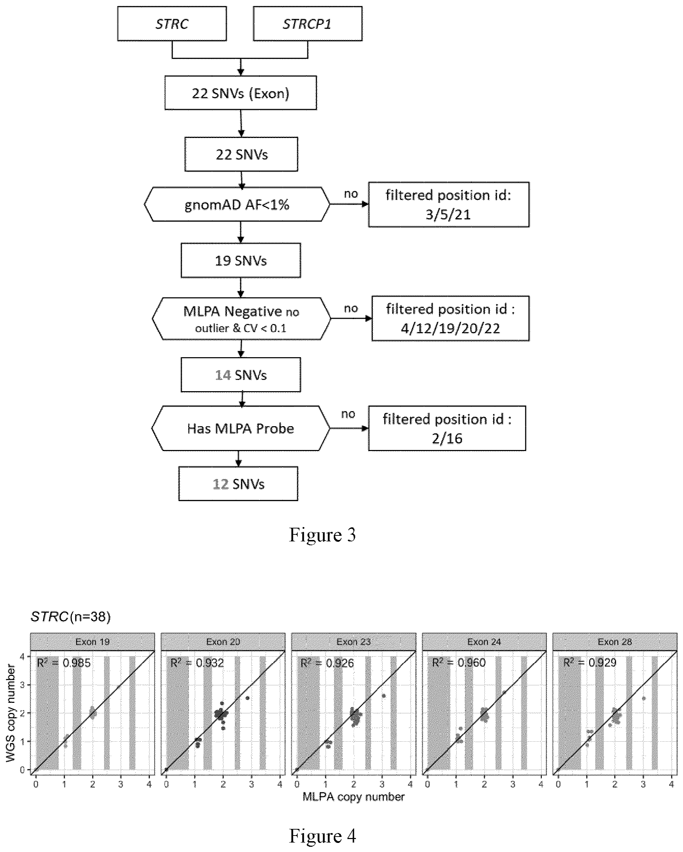

Through the sequence alignment between STRC and STRCP1 in the human reference genome, all of differentiated sites between the true gene and the pseudogene are searched out, and positions corresponding to the differentiated sites and bases at these positions are output to form a differentiated-site list of the true gene over the pseudogene. With a series of filtering conditions, differentiated sites that may negatively affect obtaining correct analysis results are removed, and the results are shown in Figure 3. Specifically, the human reference genome may be downloaded from UCSC first, and DNA sequences of STRC and STRCP1 may be extracted by BEDTools. With the sequence alignment, 22 single-base differentiated sites on exons between the true gene and pseudogene are obtained. In order to ensure that the sites used in this embodiment have general applicability in the population, those polymorphic sites with a high population frequency are removed, where the sites with population frequency greater than 1% are filtered out, by using, for example, a population frequency database gnomAD.

In addition, for the stability of the analysis, multiple, such as 27, negative samples have been validated by MLPA (multiplex ligation-dependent probe amplification) are used. Negative samples herein refer to samples on which detections with STRC probes are negative.

Further, the differentiated sites in these negative samples are calculated for a proportional distribution and evaluated for the fluctuation of these sites with a coefficient of variation (CV). Differentiated sites, for example 14 differentiated sites, with CV<0.1, are retained finally.

In addition, considering that a gold standard for copy-number variation detection of STRC gene is MLPA-probe detection at present, differentiated sites uncovered by MLPA detection may be removed, in order to render the analysis results of the method for detecting copy number variation of STRC gene based on WGS as provided by embodiments of the present disclosure comparable to the gold standard. The difference sites, for example, 12 differentiated sites, encompassed by MLPA detection are retained finally for subsequent analysis (Table 1).

Table 1: Differentiated sites between STRC and STRCP1

| gene | region | chrom | pos | ref | $\psi$chrom | $\psi$pos | $\psi$ref |
|------|--------|-------|-----|-----|---------|-------|-------|
| **STRC** | Ex28 | 15 | 43892272 | T | 15 | 43992088 | C |
| **STRC** | Ex24 | 15 | 43893602 | T | 15 | 43993420 | A |
| **STRC** | Ex24 | 15 | 43893673 | C | 15 | 43993491 | T |
| **STRC** | Ex24 | 15 | 43893681 | T | 15 | 43993499 | C |
| **STRC** | Ex24 | 15 | 43893696 | C | 15 | 43993514 | T |
| **STRC** | Ex24 | 15 | 43893702 | A | 15 | 43993520 | G |
| **STRC** | Ex24 | 15 | 43893725 | G | 15 | 43993543 | C |
| **STRC** | Ex23 | 15 | 43895520 | C | 15 | 43995030 | T |
| **STRC** | Ex23 | 15 | 43895542 | T | 15 | 43995052 | A |
| **STRC** | Ex23 | 15 | 43895560 | C | 15 | 43995070 | G |
| **STRC** | Ex20 | 15 | 43896918 | G | 15 | 43996398 | A |
| **STRC** | Ex19 | 15 | 43897597 | C | 15 | 43997075 | T |

These differentiated sites are based on positions of the GRCh37/hg19 reference, and column names in Table 1 are gene name, and then exon region, chromosome of the true gene, chromosome position of the true gene, base type of the true gene, chromosome of the pseudogene, chromosome position of the pseudogene, and base type of the pseudogene.

At step S120, for each of the differentiated sites, respective sequences at corresponding positions in the STRC gene and the STRCP1 gene in a genome are read from a variant calling file.

At step S130, a total copy number of the true gene and the pseudogene is calculated with a reference site preset in the genome as a benchmark.

In embodiments of the present disclosure, the strategy for calculating copy number of the gene includes: calculating the total copy number, Total_CN, of the true gene STRC and the pseudogene STRCP1 firstly; then calculating respective copy ratios, Ratio, of the true gene STRC and the pseudogene STRCP1 based on information of the differentiated sites between the true gene and the pseudogene; and obtaining respective copy numbers of STRC and STRCP1 by multiplying the total copy number by the respective ratios.

In calculating the total copy number of the true gene and the pseudogene, firstly, for each of the differentiated sites, respective sequences at corresponding positions in STRC and STRCP1 in genome are read from the variant calling file GVCF, the gathered base information of said corresponding positions in STRC and STRCP1 is served as a total read number of the true gene and the pseudogene. Then, with the reference site such as 2,000 reference sites preset in the genome as a benchmark, the total copy number of the true gene and the pseudogene is calculated.

In a specific embodiment, an expression for the total copy number is as follows:

$$Total\_CN_i = \frac{Median\ depth\ of\ STRC\ and\ STRCP1}{Median\ depth\ of\ control} \times 2,$$

in which *"i"* represents the *i* th differentiated site, which is a positive integer; *Total_CN$_i$* represents the total copy number of the true gene STRC and the pseudogene STRCP1 at the *i* th differentiated site; *"Median depth of STRC and STRCP1"* represents a median of depth of the true gene STRC and the pseudogene STRCP1; and *"Median depth of control"* represents a median of depth of the reference site.

At step S140, a STRC gene copy ratio for each of the differentiated sites is calculated.

In an embodiments of the present disclosure, in the step S140, calculating a STRC gene copy ratio for each of the differentiated sites includes:

S1: calculating a base number at the corresponding positions in the STRC gene and the STRCP1 gene based on the sequences at the corresponding positions in the STRC gene and the STRCP1 gene, to obtain a total number of sequences of the true gene and the pseudogene;
S2: reading from the variant calling file a first number of same bases in the corresponding position in STRC or in STRCP1 as that in the STRC gene, and denotingthe first number as a STRC-sequence count; and
S3: calculating the STRC gene copy ratio based on the total number of sequences and the STRC-sequence count.

Specifically, the STRC gene copy ratio is the number of STRC gene bases divided by the total base number (i.e. the total number of sequences) of STRC gene and STRCP1 gene, for a differentiated site. An expression for the STRC gene copy ratio is as follows:

$$STRC\_Ratio_i = \frac{STRC\ specifc\ reads}{Total\ reads\ of\ STRC\ and\ STRCP1},$$

in which *"i"* represents the *i* th differentiated site; $STRC\_Ratio_i$ represents the STRC gene copy ratio at the *i* th differentiated site; *"STRC specific reads"* represents the number of sequences of the true gene STRC at the corresponding position in the true gene STRC, which are read from the GVCF and are represented by the number of same bases in the corresponding position in STRC or s in STRCP1 as that in the STRC gene; and *"Total reads of STRC and STRCP1"* represents the total sequence number of the true gene and the pseudogene.
Further, provided in embodiments of the present disclosure is calculation of a STRCP1 gene copy ratio, including the following process.
In an embodiment, the method further includes: reading from the variant calling file a second number of same bases in the corresponding position in STRC or in STRCP1 as that in the STRCP1 gene, and denoting the second number as a STRCP1-sequence count; and calculating the STRCP1 gene copy ratio based on the total number of sequences and the STRCP1-sequence count.
Specifically, the STRCP1 gene copy ratio is the number of STRCP1 gene bases divided by the total base number (i.e. the total number of sequences) of STRC gene and STRCP1 gene, for a differentiated site. An expression for the STRCP1 gene copy ratio is as follows:

$$STRCP1\_Ratio_i = \frac{STRCP1\ specifc\ reads}{Total\ reads\ of\ STRC\ and\ STRCP1},$$

in which *"i"* represents the *i* th differentiated site; $STRCP1\_Ratio_i$ represents the STRCP1 gene copy ratio at the *i* th differentiated site; *"STRCP1 specific reads"* represents the number of sequences of the pseudogene STRCP1 at the corresponding position in the pseudogene STRCP1, which are read from the GVCF and are represented by the number of same bases in the corresponding position in STRC or in STRCP1 as that in the STRCP1 gene; and *"Total reads of STRC and STRCP1"* represents the total sequence number of the true gene and the pseudogene.
At step S150, a first STRC gene copy number for each of the differentiated sites is calculated based on the total copy number and the STRC gene copy ratio.
Based on the total copy number and the STRC gene copy ratio, the copy number of STRC gene (i.e. the true gene) is obtained. A specific expression is as follows:

$$STRC\_CN_i = Total\_CN_i \times STRC\_Ratio_i,$$

in which $STRC\_CN_i$ represents the copy number of STRC for the *i* th differentiated site.
Further, provided in embodiments of the present disclosure is calculation of a STRCP1 gene copy number, including the following process.
In an embodiment, the method further includes: calculating a first STRCP1 gene copy number for each of the differentiated sites based on the total copy number and the STRCP1 gene copy ratio.
Specifically, based on the total copy number and the STRCP1 gene copy ratio, the copy number of STRCP1 gene (i.e. the pseudogene) is obtained. A specific expression is as follows:

$$STRCP1\_CN_i = Total\_CN_i \times STRCP1\_Ratio_{i,}$$

in which $STRCP1\_CN_i$ represents the copy number of STRCP1 for the $i$ th differentiated site.

At step S160, a second STRC gene copy number for each exon is determined based on the first STRC gene copy number for each of the differentiated sites.

In an embodiment, the method further includes: determining a second STRCP1 gene copy number for each exon based on the first STRCP1 gene copy number for each of the differentiated sites.

With the calculated STRC gene copy number and STRCP1 gene copy number for each of the differentiated sites, the STRC gene copy number and STRCP1 gene copy number on each exon may be calculated respectively.

In an optional embodiment, the median of the STRC gene copy number at all of the differentiated sites located at an exon may be taken as the second STRC gene copy number on such an exon; and the median of the STRCP1 gene copy number at all of the differentiated sites located at an exon may be taken as the second STRCP1 gene copy number on such an exon.

According to embodiments of the present disclosure, the method for detecting copy number variation of STRC gene based on WGS as provided perform a sequence alignment between the STRC gene and the STRCP1 gene to search out differentiated sites; read from a variant calling file , for each of the differentiated sites, respective sequences at corresponding positions in the STRC gene and the STRCP1 gene in a genome; calculate a total copy number of the true gene and the pseudogene with a reference site preset in the genome as a benchmark; calculate a STRC gene copy ratio for each of the differentiated sites; calculate a first STRC gene copy number for each of the differentiated sites based on the total copy number and the STRC gene copy ratio; and determine a second STRC gene copy number for each exon based on the first STRC gene copy number for each of the differentiated sites..

According to embodiments of the present disclosure, the method for detecting copy number variation of STRC gene based on WGS could detect copy numbers of STRC for hereditary deafness, thereby expanding the range of genetic diseases detectable by WGS. Compared with traditional detection methods for highly homologous genes, the method is with simplified detection process, improved detection throughput, reduced detection cost and increased product competitiveness. Moreover, the method could automatically judge and output the copy number of each exon of STRC, which can be used for gene screening of healthy people or genetic etiology diagnosis of deaf patients.

Further, with the calculated second STRC gene copy number and STRCP1 gene copy number on each exon, distributions of the STRC gene copy number and STRCP1 gene copy number among the population data may be calculated, and a state of the exon may be judged with a decision threshold defined by statistical methods. Details are as follows.

In an embodiment, the method further includes: judging for each exon a state of the exon according to the second STRC gene copy number on the exon and/or the second STRCP1 gene copy number on the exon.

Optionally, the state of each exon may be classified as normal (wide-type), heterozygous deletion, homozygous deletion, heterozygous duplication, or homozygous duplication.

Next, reference will be made to judgment of the state of exon by taking the second STRC gene copy number on the exon as an example. For the copy number on each exon, the median (Mean) and standard deviation (SD) of the copy number on each exon in the population data are calculated. Then, a copy number on the exon falling within [Mean-1.96*SD, Mean+1.96*SD] is judged as a wide-type, and [0.1, Mean-1.96*SD] for heterozygous deletion, [0, 0.1] for homozygous deletion, [Mean+1.96*SD, 3.5] for heterozygous duplication, and greater than 3.5 is judged for homozygous duplication.

The method for judging a state of exon based on the second STRCP1 gene copy number is similar to that for STRC which is taken as an example, where each threshold may be determined according to actual situation. Each threshold may be the same as or different from those used in judgment of the state of exon by taking the second STRC gene copy number on the exon as an example as described above, which will be not repeated herein.

The method provided in embodiments of the present disclosure makes a judgment for exon state according to gene copy number, which significantly reduced manual cost on variation interpretation, and is suitable for gene screening of healthy people or genetic etiology diagnosis of deaf patients.

**Examples of Effect Implementation**

Example 1

In order to verify the effectiveness of the method for detecting copy number variation of STRC gene based on whole genome sequencing provided in Examples of the present disclosure, a comparison between copy numbers of STRC obtained by the detection method of the Example of the present disclosure and by a MLPA kit was performed. The specific process is as follows: 38 samples previously collected after validation by a MLPA kit as positive or negative were selected for whole genome sequencing, and were detected for STRC copy number with the above analysis protocol. A negative sample refers to MLPA-probe-test results show that the exon copy number of STRC gene is normal, otherwise it is a positive sample. These samples included 3 homozygous deletion samples, 5 heterozygous deletion samples, 2 heterozygous duplication samples, 1 homozygous and heterozygous deletion complex sample, and 27 negative samples. By comparing the results of WGS detection with those of MLPA, it was found that the results of the two were highly consistent, and a correlation coefficient R2 of each Exon is between 0.929 and 0.985, indicating that the detection method provided in Examples of the present disclosure is of accuracy and reliability; for detailed results, please refer to Figure 4, which is a correlation between WGS and MLPA results of 38 samples, where the abscissa is the copy number of STRC obtained by MLPA kit detection, and the ordinate is the copy number of STRC obtained by analyzing WGS data with the detection method provided in Examples of the present disclosure, and the correlation coefficient R2 is marked in the upper left corner of each Exon diagram.

Example 2

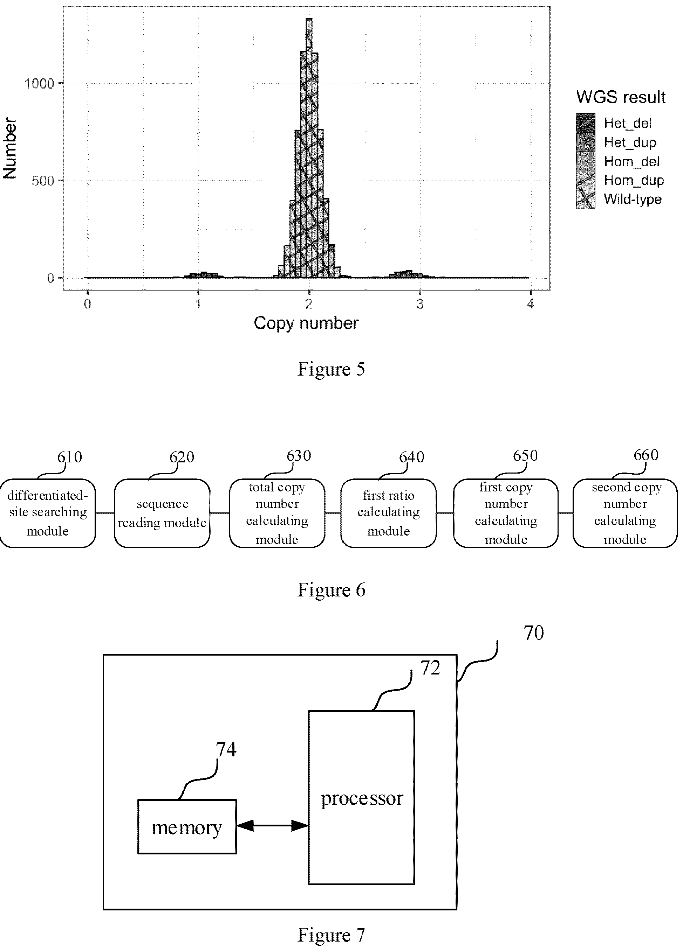

The method for detecting copy number variation of STRC gene based on whole genome sequencing provided in Examples of the present disclosure was used to analyze 9,582 whole genome sequencing samples of MGI, and the analysis results showed that there are 2 homozygous deletion samples (clinically significant as pathogenic mutation), 222 heterozygous deletion samples (clinically significant as pathogenic mutation), 5 homozygous duplication samples (unclear clinical significance), 265 heterozygous duplication samples (unclear clinical significance), and 9,088 wild-type samples (clinically insignificant) (Table 2). The copy numbers of the 5 Exons of STRC gene were combined and calculated to obtain the copy numbers in terms of STRC gene, and their distribution presented three normal distributions of heterozygous deletion, wild type and heterozygous duplication, which meet expectations; for details, please refer to Figure 5, where Het_del represents heterozygous deletion, Het_dup represents heterozygous duplication, Hom_del represents homozygous deletion, Hom_dup represents homozygous duplication, and Wild-type represents normal.

Table 2 Analysis results of STRC copy number on 2 9,582 WGS samples

| ID | genotype | clinical significance | sample cases | % |
|---|---|---|---|---|
| 0 | homozygous deletion | pathogenic mutation | 2 | 0.021 |
| 1 | heterozygous deletion | pathogenic mutation | 222 | 2.40 |
| 2 | normal | - | 9088 | 94.67 |
| 3 | homozygous duplication | unclear | 265 | 2.86 |
| 4 | heterozygous duplication | unclear | 5 | 0.052 |

It should be understood that although the various steps in the flowchart of Figure 2 are shown sequentially as indicated by the arrows, the steps are not necessarily performed as these. Unless explicitly stated herein, there is no strict order in which the steps may be performed, and the steps may be performed in other orders. Moreover, at least a part of the steps in Figure 2 may comprise a plurality of sub-steps or a plurality of stages, and these sub-steps or stages are not necessarily performed at the same time, but may be performed at different times, and the execution order of these sub-steps or stages is not necessarily performed sequentially, but may be performed in turn or alternately with at least a part of other steps or sub-steps or stages of other steps.

Examples above detail the method for detecting copy number variation of STRC gene based on whole genome sequencing, which could be implemented by various apparatuses. The Examples of the present disclosure therefore further disclose a device for detecting copy number variation of STRC gene based on whole genome sequencing corresponding to the method above. Specific Examples are described below for detailed explanation.

Referring to Figure 6 illustrating a device for detecting copy number variation of STRC gene based on whole genome sequencing according to Examples of the present disclosure, the device mainly includes:

a differentiated-site searching module 610, configured to perform a sequence alignment between the STRC gene and a STRCP1 gene to search out differentiated sites;
a sequence reading module 620, configured to read from a variant calling file , for each of the differentiated sites, respective sequences at corresponding positions in the STRC gene and the STRCP1 gene in a genome;
a total copy number calculating module 630, configured to calculate a total copy number of the true gene and the pseudogene with a reference site preset in the genome as a benchmark;
a first ratio calculating module 640, configured to calculate a STRC gene copy ratio for each of the differentiated sites;
a first copy number calculating module 650, configured to calculate a first STRC gene copy number for each of the differentiated sites based on the total copy number and the STRC gene copy ratio; and

a second copy number calculating module 660, configured to determine a second STRC gene copy number for each exon based on the first STRC gene copy number for each of the differentiated sites.

In an embodiments, the first ratio calculating module 640 is configured to calculate a base number at the corresponding positions in the STRC gene and the STRCP1 gene based on the sequences at the corresponding positions in the STRC gene and the STRCP1 gene, to obtain a total number of sequences of the true gene and the pseudogene; read from the variant calling file a first number of same bases in the corresponding position in STRC or in STRCP1 as that in the STRC gene, and denotingthe first number as a STRC-sequence count; and calculate the STRC gene copy ratio based on the total number of sequences and the STRC-sequence count.

In an embodiments, the differentiated-site searching module 610 is configured to extract from a human reference genome STRC gene and STRCP1 gene; perform the sequence alignment between STRC gene and STRCP1 gene to obtain all of initially differentiated sites between the STRC gene and the STRCP1 gene; and selecte from all of the initially differentiated sites the differentiated sites with a population frequency less than a first preset threshold and a coefficient of variation less than a second preset threshold.

In an embodiments, the device further includes: a second ratio calculating module, configured to read from the variant calling file a second number of same bases in the corresponding position in STRC or in STRCP1 as that in the STRCP1 gene, and denote the second number as a STRCP1-sequence count; and calculate the STRCP1 gene copy ratio based on the total number of sequences and the STRCP1-sequence count.

In an embodiments, the device further includes: a third copy number calculating module, configured to calculate a first STRCP1 gene copy number for each of the differentiated sites based on the total copy number and the STRCP1 gene copy ratio.

In an embodiments, the device further includes: a fourth copy number calculating module, configured to determine a second STRCP1 gene copy number for each exon based on the first STRCP1 gene copy number for each of the differentiated sites.

In an embodiments, the device further includes: a state judging module, configured to judge for each exon a state of the exon according to the second STRC gene copy number on the exon and/or the second STRCP1 gene copy number on the exon.

For specific limitations of the device for detecting copy number variation of STRC gene, please refer to the above limitations on the method, and will not be described here. Each module in the above device may be implemented in whole or in part by software, hardware, or combinations thereof. Each of the above modules may be embedded in or independent of a processor in the terminal device in a hardware form, or may be stored in a memory in the terminal device in a software form, so that the processor may invoke and execute operations corresponding to each of the above modules.

Referring to Figure 7, it shows a block diagram illustrating a terminal apparatus according to Examples of the present disclosure. The terminal apparatus 70 may be a computer. The terminal apparatus 70 according to the present disclosure may include one or more of the following components: a processor 72, a memory 74 and one or more programs, where said one or more programs may be stored in the memory 74 and are configured for being executed by said one or more processors 72, and said one or more programs are configured for executing the method for detecting copy number variation of STRC gene as described in Examples above.

The processor 72 may include one or more processing cores. The processor 72 connects various parts within the entire terminal apparatus 70 by various interfaces and lines, and performs various functions and processes data of the terminal apparatus 70 by running or executing instructions, programs, code sets, or instruction sets stored in the memory 74, and calling data stored in the memory 74. Alternatively, the processor 72 may be implemented in at least one hardware form selected from Digital Signal Processing (DSP), Field-Programmable Gate Array (FPGA), and Programmable Logic Array (PLA). The processor 72 may integrate one or a combination of a central processing unit (CPU), a graphics Processing Unit (GPU), and a modem. Among them, CPU mainly handles operating system, user interface and application programs; GPU is responsible for rendering and drawing of display content; and modem is used to handle wireless communication. It would be understood that the modem may not be integrated into the processor 72, and may be implemented by a communication chip alone.

The memory 74 may include random Access memory (RAM) or read-only memory (ROM). The memory 74 may be used to store instructions, programs, code, code sets, or instruction sets. The memory 74 may include a program storage area and a data storage area, where the program storage area may store instructions for implementing an operating system, instructions for implementing at least one function (such as a touch function, a sound playback function, an image playback function, etc.), instructions for implementing various method embodiments described below, etc. The storage data area may also store data or the like created in use by the terminal apparatus 70.

A person skilled in the art may understand that the structure shown in Figure 7 is only a block diagram of a part of the structure related to the solution of the present disclosure, and does not constitute a limitation on the terminal apparatus to which the solution of the present disclosure is applied. A specific terminal apparatus may include more or less

# EP 4 697 335 A1

components than those shown in this Figure, or combine some components, or have different component arrangements.

To sum up, the terminal apparatus provided in Examples of the present disclosure is used to implement the corresponding method for detecting copy number variation of STRC gene based on whole genome sequencing as described in Examples above, and presents beneficial effects of the corresponding method embodiment, which are not repeated here.

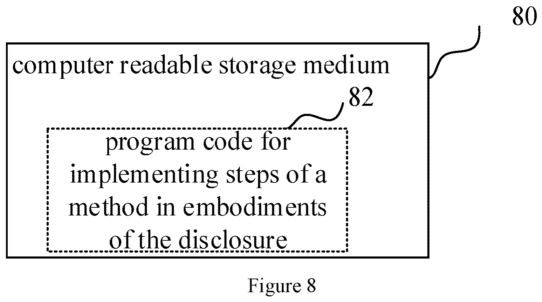

Referring to Figure 8, it shows a block diagram illustrating a computer readable storage medium according to Examples of the present disclosure. The computer readable storage medium 80 having stored therein a program code that, when executed by a processor, implements the method for detecting copy number variation of STRC gene based on whole genome sequencing as described in Examples above.

The computer readable storage medium 80 may be electronic memory such as flash memory, EEPROM (Electrically Erasable Programmable Read Only Memory), EPROM, hard disk, or ROM. Optionally, the computer-readable storage medium 80 includes a non-transitory computer-readable storage medium. The computer readable storage medium 80 has storage space for program code 82 for performing any of the method steps described above. The program code may be read from or written to one or more computer program products. The program code 82 may be compressed, for example, in an appropriate form.

Reference throughout this specification to "an embodiment", "some embodiments", "an example", "a specific example" or "some examples" means that a particular feature, structure, material, or characteristic described in connection with the embodiment or example is included in at least one embodiment or example of the present disclosure. Thus, the appearances of the phrases such as "in some embodiments", "in one embodiment", "in an embodiment", "in another example", "in an example", "in a specific example" or "in some examples", in various places throughout this specification are not necessarily referring to the same embodiment or example of the present disclosure. Furthermore, the particular features, structures, materials, or characteristics may be combined in any suitable manner in one or more embodiments or examples. Besides, any different embodiments and examples and any different characteristics of embodiments and examples may be combined by those skilled in the art without contradiction.

The above description of the disclosed embodiments enables one skilled in the art to make or use the disclosure. Various modifications to these embodiments will be apparent to those skilled in the art, and the general principles defined herein may be implemented in other embodiments without departing from the spirit or scope of the present application. Thus, the application is not intended to be limited to the embodiments shown herein but is to be accorded the widest scope consistent with the principles and novel features disclosed herein.

## Claims

1. A method for detecting copy number variation of a stereocilin (STRC) gene based on whole genome sequencing, comprising:

   performing a sequence alignment between the STRC gene and a stereocilin pseudogene 1 (STRCP1) gene to search out differentiated sites;
   reading, from a variant calling file, for each of the differentiated sites, respective sequences at corresponding positions in the STRC gene and the STRCP1 gene in a genome;
   calculating a total copy number of the true gene and the pseudogene with a reference site preset in the genome as a benchmark;
   calculating a STRC gene copy ratio for each of the differentiated sites;
   calculating a first STRC gene copy number for each of the differentiated sites based on the total copy number and the STRC gene copy ratio; and
   determining a second STRC gene copy number for each exon based on the first STRC gene copy number for each of the differentiated sites.

2. The method according to claim 1, wherein calculating a STRC gene copy ratio for each of the differentiated sites comprises:

   calculating a base number at the corresponding positions in the STRC gene and the STRCP1 gene based on the sequences at the corresponding positions in the STRC gene and the STRCP1 gene, to obtain a total number of sequences of the true gene and the pseudogene;
   reading from the variant calling file a first number of same bases in the corresponding position in the STRC gene or the STRCP1 gene as that in the STRC gene, and denoting the first number as a STRC-sequence count; and
   calculating the STRC gene copy ratio based on the total number of sequences and the STRC-sequence count.

3. The method according to claim 1, wherein performing a sequence alignment between the STRC gene and STRCP1 gene to search out differentiated sites comprises:

    extracting from a human reference genome the STRC gene and the STRCP1 gene;
    performing the sequence alignment between the STRC gene and the STRCP1 gene to obtain all of initially differentiated sites between the STRC gene and the STRCP1 gene; and
    selecting from all of the initially differentiated sites the differentiated sites with a population frequency less than a first preset threshold and a coefficient of variation less than a second preset threshold.

4. The method according to claim 2, further comprising:

    reading from the variant calling file a second number of same bases in the corresponding position in the STRC gene or the STRCP1 gene as that in the STRCP1 gene, and denoting the second number as a STRCP1-sequence count; and
    calculating a STRCP1 gene copy ratio based on the total number of sequences and the STRCP1-sequence count.

5. The method according to claim 4, further comprising:
calculating a first STRCP1 gene copy number for each of the differentiated sites based on the total copy number and the STRCP1 gene copy ratio.

6. The method according to claim 5, further comprising:
determining a second STRCP1 gene copy number for each exon based on the first STRCP1 gene copy number for each of the differentiated sites.

7. The method according to claim 6, further comprising:
judging for each exon a state of the exon according to the second STRC gene copy number on the exon and/or the second STRCP1 gene copy number on the exon.

8. A device for detecting copy number variation of a STRC gene based on whole genome sequencing, comprising:

    a differentiated-site searching module, configured to perform a sequence alignment between the STRC gene and a STRCP1 gene to search out differentiated sites;
    a sequence reading module, configured to read from a variant calling file , for each of the differentiated sites, respective sequences at corresponding positions in the STRC gene and the STRCP1 gene in a genome;
    a total copy number calculating module, configured to calculate a total copy number of the true gene and the pseudogene with a reference site preset in the genome as a benchmark;
    a first ratio calculating module, configured to calculate a STRC gene copy ratio for each of the differentiated sites;
    a first copy number calculating module, configured to calculate a first STRC gene copy number for each of the differentiated sites based on the total copy number and the STRC gene copy ratio; and
    a second copy number calculating module, configured to determine a second STRC gene copy number for each exon based on the first STRC gene copy number for each of the differentiated sites.

9. A terminal apparatus, comprising:

    one or more processors; and
    a memory, for storing one or more programs executable by said one or more processors;
    wherein said one or more processors are coupled with the memory, and said one or more programs is configured to execute the method according to any one of claims 1 to 7.

10. A computer readable storage medium having stored therein a program code that, when executed by a processor, implements the method according to any one of claims 1 to 7.

Figure 1

performing a sequence alignment between a STRC gene and a STRCP1 gene to search out differentiated sites — S110

reading, from a variant calling file, for each of the differentiated sites, respective sequences at corresponding positions in the STRC gene and the STRCP1 gene in a genome — S120

calculating a total copy number of the true gene and the pseudo gene with a reference site preset in the genome as a benchmark — S130

calculating a STRC gene copy ratio for each of the differentiated sites — S140

calculating a first STRC gene copy number for each of the differentiated sites based on the total copy number and the STRC gene copy ratio — S150

determining a second STRC gene copy number for each exon based on the first STRC gene copy number for each of the differentiated sites — S160

Figure 2

Figure 3

Figure 4

Figure 5

Figure 6

Figure 7

80

computer readable storage medium

82

program code for
implementing steps of a
method in embodiments
of the disclosure

Figure 8

## INTERNATIONAL SEARCH REPORT

| International application No. |
| --- |
| **PCT/CN2023/096747** |

**A. CLASSIFICATION OF SUBJECT MATTER**

G16B20/10(2019.01)i; G16B30/10(2019.01)i

According to International Patent Classification (IPC) or to both national classification and IPC

**B. FIELDS SEARCHED**

Minimum documentation searched (classification system followed by classification symbols)

IPC:G16B,C12Q

Documentation searched other than minimum documentation to the extent that such documents are included in the fields searched

Electronic data base consulted during the international search (name of data base and, where practicable, search terms used)

DWPI, CNABS, WPABS, CNTXT, ENTXT, CNKI, VEN: 高通量测序, 聋, 耳聋, 拷贝数, 检测, 差异, 变异, 失聪, 点位, 位点, 基因, 外显子, High-throughput sequencing, deafness, deafness, copy number, detection, difference, variation, deafness, site, locus, gene, exon, STRC, STRCP1.

**C. DOCUMENTS CONSIDERED TO BE RELEVANT**

| Category* | Citation of document, with indication, where appropriate, of the relevant passages | Relevant to claim No. |
| --- | --- | --- |
| PX | CN 116453588 A (SHENZHEN BGI GENOMICS CO., LTD.) 18 July 2023 (2023-07-18) claims 1-10 | 1-10 |
| A | CN 112201306 A (GUANGZHOU KINGMED DIAGNOSTICS GROUP CO., LTD.; GUANGZHOU KINGMED CENTER FOR CLINICAL LABORATORY CO., LTD.; KINGMED DIAGNOSTICS (HONG KONG) LIMITED) 08 January 2021 (2021-01-08) entire document | 1-10 |
| A | CN 113724791 A (BGI TIANJIN; BGI SHENZHEN CO., LTD.) 30 November 2021 (2021-11-30) entire document | 1-10 |
| A | WO 2022240762 A1 (UNIVERSITY OF IOWA RESEARCH FOUNDATION) 17 November 2022 (2022-11-17) entire document | 1-10 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **21 December 2023** | **26 December 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2023/096747**

### C. DOCUMENTS CONSIDERED TO BE RELEVANT

| Category* | Citation of document, with indication, where appropriate, of the relevant passages | Relevant to claim No. |
|---|---|---|
| A | CN 115762630 A (GUANGZHOU HUAYIN MEDICAL LABORATORY CENTER CO., LTD.; GUANGZHOU HUAYIN HEALTHCARE GROUP CO., LTD.) 07 March 2023 (2023-03-07) <br> entire document | 1-10 |
| A | US 2009098547 A1 (AFFYMETRIX, INC.) 16 April 2009 (2009-04-16) <br> entire document | 1-10 |
| A | US 2014038830 A1 (VERINATA HEALTH, INC.) 06 February 2014 (2014-02-06) <br> entire document | 1-10 |
| A | WO 2018144449 A1 (COUNSYL, INC.) 09 August 2018 (2018-08-09) <br> entire document | 1-10 |

**INTERNATIONAL SEARCH REPORT**
Information on patent family members

International application No.

**PCT/CN2023/096747**

| Patent document cited in search report | | | Publication date (day/month/year) | Patent family member(s) | | | Publication date (day/month/year) |
|---|---|---|---|---|---|---|---|
| CN | 116453588 | A | 18 July 2023 | None | | | |
| CN | 112201306 | A | 08 January 2021 | None | | | |
| CN | 113724791 | A | 30 November 2021 | None | | | |
| WO | 2022240762 | A1 | 17 November 2022 | None | | | |
| CN | 115762630 | A | 07 March 2023 | None | | | |
| US | 2009098547 | A1 | 16 April 2009 | US | 2019287650 | A1 | 19 September 2019 |
| | | | | US | 11657900 | B2 | 23 May 2023 |
| | | | | US | 10229244 | B2 | 12 March 2019 |
| US | 2014038830 | A1 | 06 February 2014 | WO | 2014015319 | A1 | 23 January 2014 |
| | | | | US | 9411937 | B2 | 09 August 2016 |
| WO | 2018144449 | A1 | 09 August 2018 | US | 2018237845 | A1 | 23 August 2018 |

Form PCT/ISA/210 (patent family annex) (July 2022)

**EP 4 697 335 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310439706